# EUROPEAN PATENT SPECIFICATION

(11) **EP 1 989 193 B1**
(45) Date of publication and mention of the grant of the patent: **22.06.2011**
(21) Application number: 07711690.3
(22) Date of filing: 27.02.2007
(51) Int. Cl.: C07D 333/12, C07D 333/14, C07D 333/24

(54) **PROCESS FOR OBTAINING ENANTIOMERS OF DULOXETINE PRECURSORS**
VERFAHREN ZUR GEWINNUNG VON ENANTIOMEREN VON DULOXETIN-VORSTUFEN
PROCEDE D'OBTENTION D'ENANTIOMERES DE PRECURSEURS DE LA DULOXETINE

(30) Priority: 28.02.2006 EP 06380038
(43) Date of publication of application: 12.11.2008
(73) Proprietor: Laboratorios del. Dr. Esteve, S.A., 08041 Barcelona (ES)
(72) Inventor: BUSCHMANN, Helmut Heinrich, E-08960 San Just Desvern - Barcelona (ES); TORRENS JOVER, Antoni, E-08221 Terrasa - Barcelona (ES); LORMANN, Matthias, 52072 Aachen (DE); DAHMEN, Stefan, 67251 Freinsheim (DE)
(74) Representative: ABG Patentes, S.L.
(86) International application number: PCT/EP2007/001674
(87) International publication number: WO 2007/098923

(56) References cited:
- DE-A1- 10 237 272
- BETTE ET AL.: "[Zinc-Diamine]-Catalyzed Hydrosilylation of Ketones in Methanol. New Developments and Mechanistic Insights" ADV. SYNTH. CATAL., vol. 347, 2005, pages 289-302, XP002387638

## Description

### FIELD OF THE INVENTION

The present invention relates to a new process for the preparation of enantiomerically enriched thienylalcoxypropaneamines. Said compounds are useful intermediates for the preparation of Duloxetine.

### BACKGROUND OF THE INVENTION

The compound (±)-N-Methyl-N-[3-(naphthalene-1-yloxy)-3-(2-thienyl)propyl]amine, also known as Duloxetine, of formula I 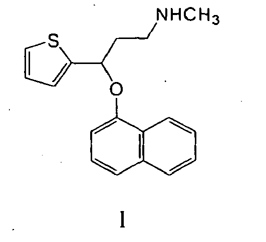 is a potent 5-HT reuptake and norepinephrine reuptake inhibitor which has been already launched to the market for the treatment of different conditions (depression, urinary incontinence and neuropathic pain). Duloxetine is also under clinical trials for the treatment of other conditions such as generalized anxiety and fibromyalgia. Of the two possible enantiomers of Duloxetine, the dextrorotatory enantiomer, (+)-Duloxetine, is more potent than the (-)-Duloxetine.

Duloxetine can be prepared by O-alkylation of a compound of formula II:

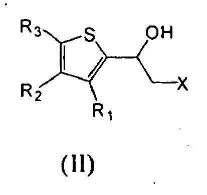

The pure enantiomers of (+)-1 and (-)-1 may be prepared by separately O-alkylating the enantiomerically pure intermediates (+)-II and (-)-II. Thus, a synthetic process to the enantiomerically pure/enriched intermediates (+)-I and (-)-II is needed.

The enantioselective reduction of prochiral ketones has been proposed in organic synthesis to obtain secondary alcohols with high enantiomeric purity. Accordingly, a number of strategies for the asymmetric reduction of prochiral ketones to enantiomerically enriched alcohols have been developed [R. Noyori, T. Ohkuma, Angew. Chem. Int. Ed., 2001, 40, 40-73, Wiley-VCH Verlag].

Also, in patent application n° CA 2498756 compounds of formula II are synthesized by acylating an acetyl thiophene of formula (I) 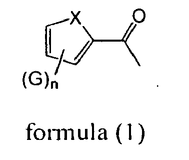 and subsequent asymmetric hydrogenation in the presence of a chiral catalyst. However, this method requires two steps in order to arrive at the compound of formula II. Additionally, the reagents needed (for example, NaH) are not compatible with industrial production.

In patent application n° DE 10237272 a thiophenecarbaldehyde derivative is subjected to a Reformatsky type reaction. A direct reduction promoted with a catalyst combining zinc and a diamine ligand has been proposed [V. Bette, A: Mortreux, D: Savoia, F:F: Carpentier, Adv. Synth. Catal. 2005, 347, 289-302].

A phenyl transfer reaction to aryl aldehydes as an approach towards enantio-pure diarylalcohols has also been proposed, as alternative to the enantioselective reduction of prochiral ketones [P.I. Dosa, J.C. Ruble, G.C. Fu, J. Org. Chem. 1997, 62 444; W.S. Huang, L. Pu, Tetrahedron Lett. 2000, 41, 145; M. Fontes, X.Verdaguer, L. Solà, M.A. Pericàs, A. Riera, J. Org. Chem. 2004, 69, 2532]. For this transformation, the group of Bolm et al. developed a protocol which utilized a ferrocene-based ligand (or catalyst) and diphenylzinc in combination with diethylzinc as aryl source [C. Bolm, N. Hermanns, M. Kesselgruber, J.P. Hildebrand, J. Organomet. Chem. 2001, 624, 157; C. Bolm, N. Hermanns, A. Claβen, K. Muniz, Bioorg. Med. Chem. Lett. 2002, 12,1795].. Enantiomerically enriched diarylmethanols with excellent enantiomeric excess (up to 99% ee) were thus obtained in a straightforward manner. Subsequently, the applicability of air-stable arylboronic acids as aryl source was also demonstrated [C. Bolm, J. Rudolph, J. Am. Chem. Soc. 2002, 124, 14850]. However; these systems require a high catalyst loading (of commonly 10% mol.) to achieve that high enantioselectivity. With the aim of reducing this problem, recently, it has been proposed the use of triphenylborane as an alternative phenyl source in a reaction where the ferrocene-based catalyst is also used (J. Rudolph, F. Schmidt, C. Bolm, Adv. Synth. Catal. 2004, 346, 867). It has been applied with difficulties to heteroaromatic aldehydes, for example to 2-thiophenecarbaldehyde.

However, there are still some difficulties to obtain chiral alcohols with a high yield and enantioselectivity without a high amount of catalyst. For their large-scale preparation, the application of highly efficient catalytic system and enantioselective methods employing inexpensive starting materials and simple purification steps would be most desirable.

Additionally, the application of these processes so as to transfer a heteroaryl moiety to an aldehyde is challenging. Most examples described in the literature are directed to the phenyl transfer to a benzaldehyde derivative or to an alkyl-aldehyde derivative. There is at the present time no example of an enantioselective addition of thienyl reagents to β-substituted aldehydes.

Thus, to attain satisfactory ee values by an enantioselective addition reaction, an appropriate coordination of the catalyst system and the aldehyde is required. The results with unusual substrates cannot be predicted and each addition has to be investigated separately with regard to the substrate.

### SUMMARY OF THE INVENTION

We have now surprisingly found that β-substituted aldehydes can be successfully used as substrates for a thienyl transfer reaction. We have therefore applied this process to the synthesis of the enantiomerically pure intermediates of compounds of formula II and to a process to obtain N-Methyl-N-[3-(naphthalene-1-yloxy)-3-(2-thienyl)propyl]amine and in general to thienylalcoxypropaneamines and their enantiomers. This process is specially useful for the industrial scale; it provides high enantiomeric excess with less quantity of catalyst. Additionally, raw materials are costs-effective. Further, heavy metals are not used, avoiding the presence of potentially toxic impurities. Another advantage is that impurities generated during the process are easily eliminated.

Accordingly, the present invention refers to a process for the asymmetric addition of a thienyl group to a β-substituted aldehyde by means of a thienyl zinc reagent in the presence of a chiral ligand. Said process allows the preparation of known intermediates of formula (II), which thereafter can yield, by O-alkylation, the desired enantiomers of pharmaceutically active thienylalcoxypropaneamines, particularly the pharmaceutically active compound N-Methyl-N-[3-(naphthalene-1-yloxy)-3-(2-thienyl)propyl]amine.

Therefore, according to one aspect, the present invention is directed to a process for the preparation of an enantiomerically enriched compound of formula II 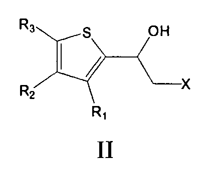 wherein,
R₁, R₂ and R₃ are each independently selected from hydrogen, halogen, lower alkyl optionally substituted by halo, hydroxy, alkoxy, alkylloxymethyl ethers, carboxy, cyano, carbonyl, acyl, alkoxycarbonyl, amino, nitro, mercapto or alkylthio; or aryl optionally substituted by hydroxy, mercapto, halo, alkyl, phenyl, alkoxy, haloalkyl, nitro, cyano, dialkylamino, aminoalkyl, acyl or alkoxycarbonyl;
X is -C(=O)-Z or Y, wherein Y is selected from -CH₂-OR₄, -CH₂-halogen or - CH₂-NR₆R₇;
wherein
Z is selected from -NR₆R₇ or -OR₅, wherein R₅ is selected from hydrogen, lower alkyl optionally substituted by halo, hydroxy, alkoxy, alkyloxymethyl ethers, carboxy, cyano, carbonyl, acyl, alkoxycarbonyl, amino, nitro, mercapto or alkylthio; or ester activating group;
R₄ is selected from hydrogen, hydroxyl protecting group or hydroxyl activating group;
R₆ and R₇ are each independently selected from hydrogen, amino protecting group, amido protecting group or lower alkyl optionally substituted by halo, hydroxy, alkoxy, alkyloxymethyl ethers, carboxy, cyano, carbonyl, acyl, alkoxycarbonyl, amino, nitro, mercapto or alkylthio;
or a pharmaceutically acceptable salt, complex or solvate thereof;
which comprises an enantioselective addition reaction to a compound of formula III 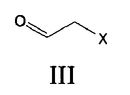 wherein X has the same meaning as above;
with a thienyl zinc reagent optionally substituted at 2, 3, 4 or 5 position of the thienyl ring by an halogen, a lower alkyl or an aryl group, in the presence of a chiral ligand.

In a preferred embodiment the present invention is directed to a process for the preparation of an enantiomerically enriched compound of formula IIa 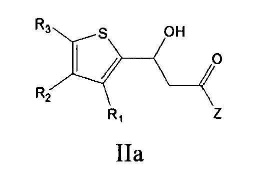 wherein,
R₁, R₂ and R₃ and Z have the same meaning as above,
or a pharmaceutically acceptable salt, complex or solvate thereof;
which comprises an enantioselective addition reaction to a compound of formula IIIa 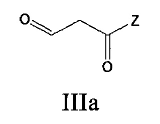 wherein Z has the same meaning as above;
with a thienyl zinc reagent optionally substituted at 2, 3, 4 or 5 position of the thienyl ring by an halogen, a lower alkyl or an aryl group, in the presence of a chiral ligand.

In another preferred embodiment the present invention is directed to a process for the preparation of an enantiomerically enriched compound of formula IIb 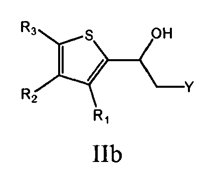 wherein,
R₁, R₂ and R₃ and Y have the same meaning as above;
or a pharmaceutically acceptable salt, complex or solvate thereof;
which comprises an enantioselective addition reaction to a compound of formula IIIb 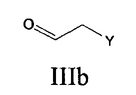
wherein Y has the same meaning as above;
with a thienyl zinc reagent optionally substituted at 2, 3, 4 or 5 position of the thienyl ring by an halogen, a lower alkyl or an aryl group, in the presence of a chiral ligand.

According to a further aspect, the present invention is directed to a process for the preparation of an enantiomerically enriched compound of formula V 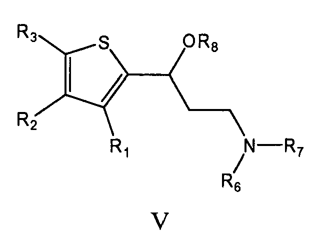 wherein
R₁, R₂, R₃, R₆ and R₇ are as defined above;
R₈ is selected from hydrogen, lower alkyl optionally substituted by halo, hydroxy, alkoxy, alkyloxymethyl ethers, carboxyl, cyano, carbonyl, acyl, alkoxycarbonyl, amino, nitro, mercapto or alkylthio; hydroxyl protecting groups; or aryl optionally substituted by hydroxy, mercapto, halo, alkyl, phenyl, alkoxy, haloalkyl, nitro, cyano, dialkylamino, aminoalkyl, acyl or alkoxycarbonyl;
or a pharmaceutically acceptable salt, complex or solvate thereof,
which comprises,
a) an enantioselective addition reaction to a compound of formula III 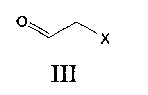 wherein X has the same meaning as above;
   of a thienyl zinc reagent optionally susbtituted at 2, 3, 4, or 5 position of the thienyl ring by an halogen, a lower alkyl or an aryl group, in the presence of a chiral ligand to yield a compound of formula II 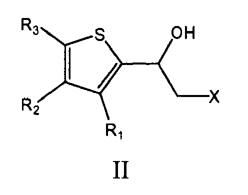 wherein,
   R₁, R₂, R₃ and X are as defined above;
   and further comprises, in any order, one or more of the steps selected from the group consisting of:
b) alkylation of the hydroxyl group;
c) amide formation;
d) reduction of amide to amine;
e) nucleophilic substitution;
e) hydroxyl, amide or amine protection;
f) hydroxyl , amide or amine deprotection;
g) salt, complex or solvate formation.

### DETAILED DESCRIPTION OF THE INVENTION

According to one aspect, the present invention relates to a process for the preparation of an enantiomerically enriched compound of formula II 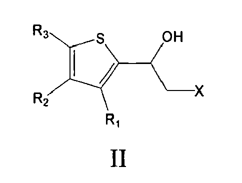 wherein,
R₁, R₂ and R₃ are each independently selected from hydrogen, halogen, lower alkyl optionally substituted by halo, hydroxy, alkoxy, alkyloxymethyl ethers, carboxy, cyano, carbonyl, alkoxylcarbonyl, amino, nitro, mercapto or alkylthio; or aryl optionally substituted by hydroxy, mercapto, halo, alkyl, phenyl, alkoxy, haloalkyl, nitro, cyano, dialkylamino, aminoalkyl, acyl or alkoxycarbonyl;
X is -C(=O)-Z or Y, wherein Y is selected from -CH₂-OR₄, -CH₂-halogen or - CH₂-NR₆R₇;
wherein
Z is selected from -NR₆R₇ or -OR₅, wherein R₅ is selected from hydrogen, lower alkyl optionally substituted by halo, hydroxy, alkoxy, alkyloxymethyl ethers, carboxy, cyano, carbonyl, acyl, alkoxylcarbonyl, amino, nitro, mercapto or alkylthio; or ester activating group;
R₄ is selected from hydrogen, hydroxyl protecting group or hydroxyl activating group;
R₆ and R₇ are each independently selected from hydrogen, amino protecting group, amido protecting group or lower alkyl optionally substituted by halo, hydroxy, alkoxy, alkyloxymethyl ether, carboxyl, cyano, carbonyl, acyl alkoxycarbonyl, amino, nitro, mercapto or alkylthio;
or a pharmaceutically acceptable salt, complex or solvate thereof;
which comprises an enantioselective addition reaction to a compound of formula III 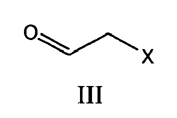 wherein X has the same meaning as above,
with a thienyl zinc reagent optionally substituted at 2, 3, 4,or 5 position of the thienyl ring by an halogen, a lower alkyl or an aryl group, in the presence of a chiral ligand.

Such a process gives the desired products of formula II with a high conversion and enantiomeric excess. This process has the further advantage that the zinc salts used or formed during the reaction are easily removed by aqueous work-up. The product of formula II is especially useful in the preparation of the enantiomers of the above mentioned thienylalcoxypropaneamines. Different compounds can be obtained depending on the substituents present on the thienyl ring.

The term "pharmaceutically acceptable salts" refers to any salt, which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, can be carried out by methods known in the art.

For instance, pharmaceutically acceptable salts of compounds provided herein may be acid addition salts, base addition salts or metallic salts, and they can be synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium; aluminium and lithium salts.

The term "solvate'' according to this invention is to be understood as meaning any form of the active compound according to the invention which has another molecule (most likely a polar solvent) attached to it via non-covalent bonding. Examples of solvates include hydrates and alcoholates, e.g. methanolate.

"Complex" refers to a molecule which is formed by two components: a donor and an acceptor. Bonding between both components to form the complex is possible because the donor may donate an unshared pair of electrons or electrons on π orbital, which the acceptor can accommodate. In a complex more than one donor and/or more than one acceptor are possible. Also, in the same "complex" one donor may be bonded to more than one acceptor and vice versa. Besides the donor-acceptor interactions described above, other types of bonding know to the skilled person, such as covalent bonding, may exist between the donor and the acceptor.

We will discuss below the different reagents and conditions for the process of the invention.

### β-Substitutedaldehyde

The synthesis of β-substituted aldehydes of formula III which is the essential starting material for the synthesis according to the present invention, is well known to the person skilled in the art. For example, a compound of formula III wherein X is an ester group can be easily prepared through the methods described in US 4,749;811 or in Sato, Masayuki; et al. Synthesis, 1986, 8, 672-4.

A compound of formula III wherein X is -CH₂-OR₄, -CH₂-halogen or -CH₂-NR₆R₇, can be easily prepared through the methods described in Niederhauser, Andreas; et al, Helvetica Chimica Acta, 1973, 56(4), 1318-30. Synthesis, 7, 977-979; 1998. Angew. Chemie, International Edition in English, 6(5), 423-34; 1967. J. Org. Chem., 1997, 62(9), 2786-2797; J. Chem. Soc, Chemical Commu., (21), 1991, 1559-60.

### Thienyl zinc reagent

The thienylzinc reagent can be *prepared in situ* by a transmetallation reaction of a thienylboron reagent with dimethyl- or diethyl-zinc. Diethyl-zinc gives good results, although dimethyl-zinc is less prone to give alkylation reaction by-products in the reaction mixture with the aldehyde. The active species are presumably a mixed thienyl-ethyl-zinc or thienyl-methyl-zinc.

Among the suitable thienyl-boron reagents, thienylboronic acid, trithienylborane or 2-aminoethyl dithienylborinate depicted below: 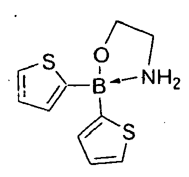 are preferably selected. More preferably, the thienyl-boron reagent is 2-aminoethyl dithienylborinate, because it can be made in higher purity and can be recrystallised from ethanol. Stable complexes of thienyl boranes are also preferred such as the NH₃ complex.

The thienyl zinc can optionally have a R₁, R₂ and/or R₃ substituent as defined above for a compound of formula II. In the process of the invention it is understood that the thienyl zinc reagent has the necessary substituents (R₁, R₂ and/or R₃) to provide directly the desired product of formula (II).

### Chiral Ligand

With the aim of enantioselectively synthesizing a compound of formula (II) by an enantioselective addition reaction, the addition reaction must be carried out in the presence of a chiral catalyst or ligand, which forms the active catalyst *in situ* by reaction with the zinc reagent. That means that the ligand (or catalyst) must have at least one element of chirality such as one or more stereocentres or elements of planar chirality.

In principle, there is a great variety of N,O-, N,N-, N,S-, N,Se- or O,O-ligands that can be used in the process of the invention and all of them have to be in enantiomerically pure form. There are in the art about 600 known ligands for this type of reaction. Most of them can be found, for example in a recent review on catalytic asymmetric organozinc additions to carbonyl compounds [L. Pu, H.-B. Yu, Chem. Rev. 2001, 101, 757]. The nomenclature N,O-, N,N-, N,S-, N,Se- or O,O- refers to ligands that have at least these two coordinating heteroatoms.

It is obvious to the skilled person that in order to obtain a compound of formula II with the opposite configuration it is only necessary to use the enantiomer of the chiral ligands used in the present invention. Therefore, when a structure of a ligand is given throughout the present patent application, said structure is also meant to encompass the enantiomer of said ligand.

In a preferred embodiment of the present invention N,O-ligands and N,S-ligands are employed.

N,O-ligands are derived from β-amino alcohols and therefore have two carbon atoms between the heteroatoms. However, some of the ligands used in this reaction are those which present three carbon atoms between the heteroatoms. More preferably, the O is an alcohol.

In one embodiment it is used a N,O-ligand having a structure-type (V) such as described below: 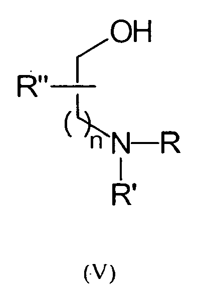 wherein
n is 0 or 1;
R and R' are independently selected from alkyl, - aryl, arylalkyl, heteroaryl and heterocyclic; and
R" is selected from the group consisting of alkyl, aryl, arylalkyl, heteroaryl and heterocyclic; or R" is a group attached to more than one carbon atom forming an aryl group.

These ligands react with the zinc reagent forming a zinc-alcoxide complex which is more Lewis-acidic than the other present zinc species (reagent and product). Additionally, it is a Lewis-base catalyst (usually at the oxygen or sulfur atom). This zinc-alcoxide complex formed in situ is the active catalyst.

Typical ligands to be used in this addition reaction are the following compounds; their enantiomers, or derivatives thereof:

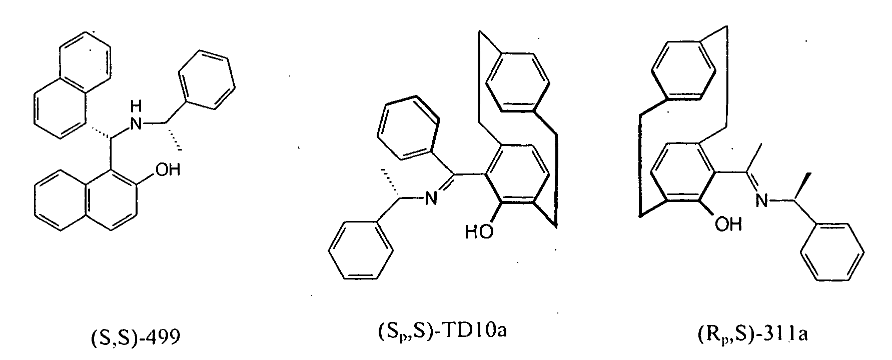

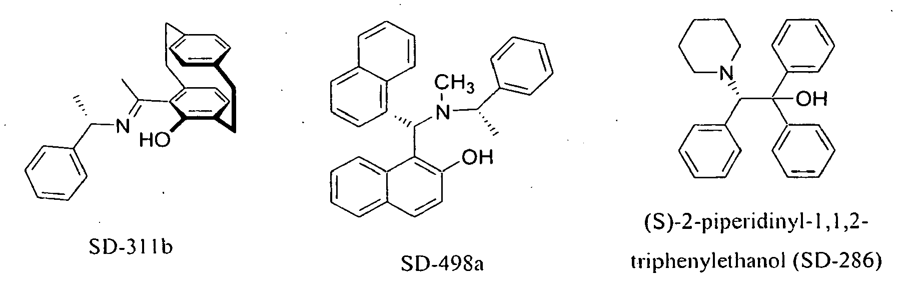

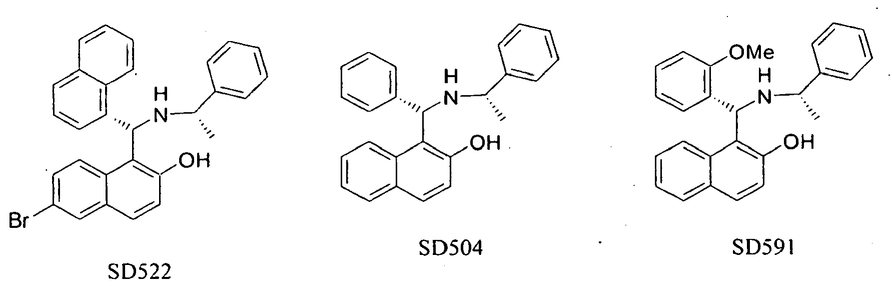

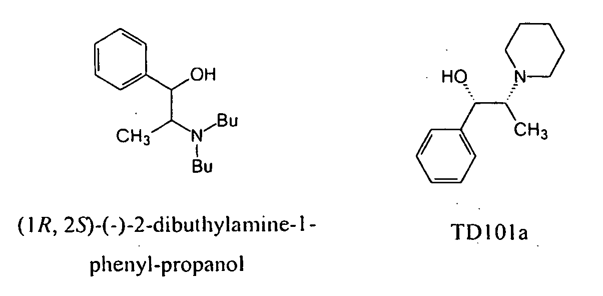

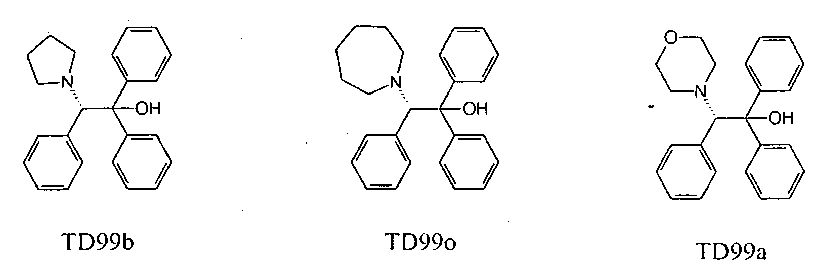

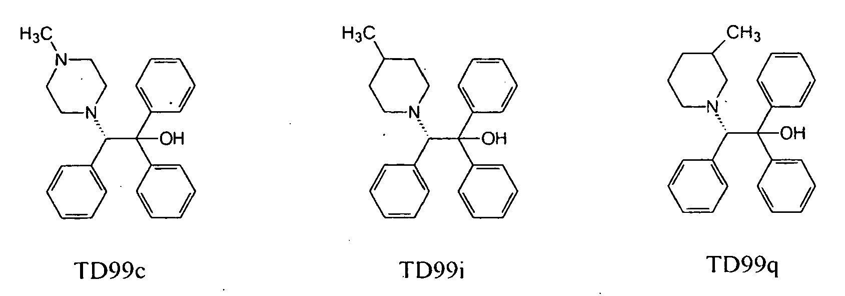

These ligands are available in both enantiomeric forms, allowing the selective synthesis of both enantiomers of the desired alcohol.

According to a further embodiment, the ligands are N,S- ligands, preferably selected from the group consisting of 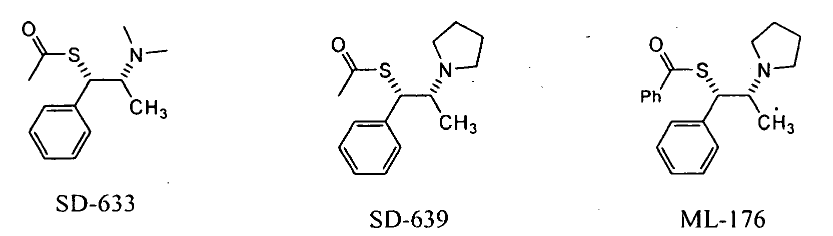 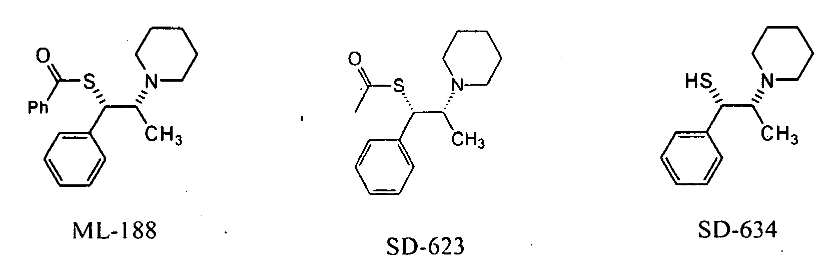 their enantiomers or derivates thereof.

N,S-ligands provided the desired product in good yield and remarkable enantiomeric excess. Among the N,S-ligands, those of formula VI 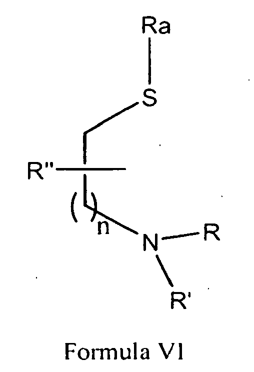 wherein n, R, R' and R'' are as defined above, and Ra is selected from hydrogen or an alkoyl group, such as the aminothioacetates, are preferred. Ligands of formula VII are readily available through known procedures (see J. Kang, J. W. Lee, J. Kim, Chem. Commun. 1994, 17, 2009 or M.-J. Jin, S.-J. Ahn, K.-S. Lee, Tetrahedron Lett. 1996, 37, 8767.)

The reaction that takes place between the zinc reagent and the ligand of formula V leads to a complex of formula (VII): 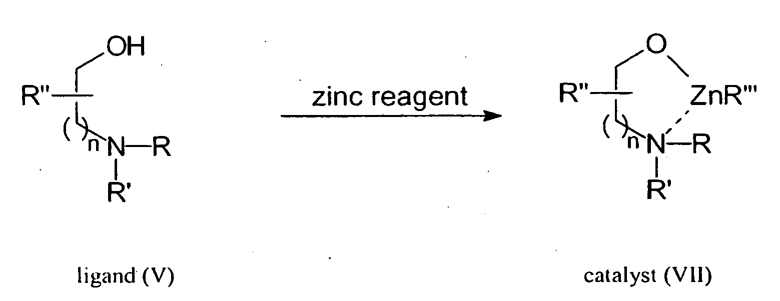 wherein n, R, R' and R" are as defined above, and R"' is thienyl, ethyl or methyl. A sulphur atom can be used instead of the oxygen atom, for example when using SD-623.

This zinc alkoxide complex (VII) is the active catalyst in the addition reaction which subsequently coordinates with the β-substitutedaldehyde in such a way that it induces the enantioselective addition of the thionyl group to said aldehyde. Without being bound by theory, it is believed that aminothiols and aminothioesters form similar complexes. However, the mechanism followed by aminothioesters complexes seems to be different from the mechanism followed by the intermediates of formula VII.

The concentration of the ligand should be low so as to reduce costs, but sufficient to provide good enantiomeric excess (ee). The ligands are preferably used in amounts of 0.1 to 100 mol%, more preferably 0.1 to 20 mol %. The use of more than the optimal amount of ligand is uneconomical and in some cases can lead to a lower selectivity. On the contrary, using less than optimal amount of ligand diminishes the selectivity due to a stronger influence of the non-catalysed and non-enantioselective background reaction.

### Solvent

Suitable solvents for the process of the invention are known from similar reactions and can be found in the above-mentioned references. Preferably they are non-coordinating hydrocarbons like e.g. pentane, hexane, heptane; aromatic solvents like benzene, toluene; chlorinated solvents like dichloromethane and 1,2-dichloroethane and weakly coordinating solvents selected from diethyl ether, methyl-*tert*-butyl ether (MTBE) and even polar coordinating solvents such as tiophene or dioxane. The most preferred solvents are toluene, hexane and heptane. These solvents allow the subsequent optional O-alkylation to be carried out in the same reaction mixture. In another variant of the process of the invention tiophene is used as the solvent, drastically improving yield and enantioselectivity and suppressing side-reactions such as the ethylation.

To perform the process, a mixture of the ligand and the compounds that form the zinc reagent can be prepared and stirred before the addition of the aldehyde. Usually, a pre-stirring is presumed to be beneficial for the selectivity because the deprotonation of the ligand by the zinc reagent giving the active catalyst requires a certain amount of time. However, under certain reaction conditions, it has been found that higher enantiomeric excess is achieved if short pre-stirring times are used. Once the aldehyde is added to the mixture of ligand and zinc reagent, the reaction time ranges between 1 h and 24 h.

The concentration of the aldehyde in the reaction is preferably low, such as between 0.01 molar and 2 molar. Although in some cases it has been observed that enantioselectivity increases at less concentrations, this is not suitable for an industrial process. In these cases a proper balance between enantioselectivity and adequate concentrations has to be found.

The process of the invention can be carried out at temperatures between -40 and 100°C. Preferably, temperatures between -20 and 20°C are used. The enantioselectivity of the reaction can also be dependent on the reaction temperature.

The process of the invention can also comprise the presence of additives, for example in order to improve the enantioselectivity by scavenging or complexing Lewis-acidic zinc salts present in the reaction or formed as products.

Suitable additives are for example alcohols, amines and derivatives of polyethylenglycol. More preferably the additive is selected from polyethylenglycols such as DiMPEG 1000, DiMPEG 2000, PEG 750, PEG 1000, PEG 2000, monoMPEG 2000 and PE-block-PEG, or from compounds such as 1,4-dioxane, *i*-propanol, triethylamine, tretramethylethylenediamine (TMEDA), imidazol, anisole, furane and thiophene. As mentioned above, the tiophene has the advantage of improving yield and enantioselectivity of the reaction, and can be used in quantities such that it acts also as a solvent.

In another aspect, the enantiomeric excess of the prepared compounds, according to the process of the present invention is enhanced by chiral HPLC and/or crystallization in appropriate solvents.

In a preferred embodiment the process is directed to the synthesis of each of the following alcohols of formula II with the highest possible enantiomeric purity: 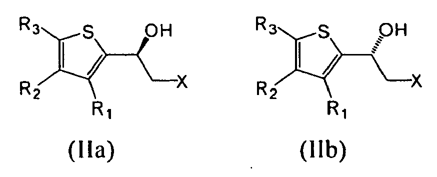 wherein R₁, R₂ and R₃ are as defined above.

The zinc salts used are easily removed by aqueous work-up and the obtained alcohol can be purified through chromatography or crystallization. Alternatively, the alcohol can be advantageously used without further purification in the next step, which can be carried out in the same reaction medium.

Thus, according to another aspect, the invention relates to a process as defined above which further comprises the step of O-alkylation of an enantiomerically enriched compound of formula (II). Preferably, oxygen is alkylated with a naphtalene derivative of formula IV 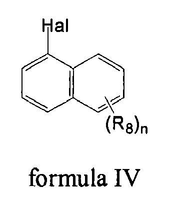 wherein Hal is F, Cl, Br or I; R₈ is lower alkyl optionally substituted by halo, hydroxy, alkoxy, alkyloxymethyl, ethers, carboxy, cyano, carbonyl, acyl, alkoxycarbonyl amino, nitro, mercapto or alkylthio; or aryl optionally substituted by hydroxy, mercapto, halo, alkyl, phenyl, alkoxy, haloalkyl, nitro, cyano, dialkylamino, aminoalkyl, acyl or alkoxycarbonyl; and n is comprised between 0 and 7. This process has been described in the art for the synthesis of duloxetine. The most frequently used method is the alkylation of the alcohol with 1-fluoronaphtalene in the presence of strong base (see EP 654264 or EP 650965, for example).

According to a preferred embodiment the O-alkylation is carried out on the product of the process of the invention without an intermediate separation or purification step.

The alkylation is preferably carried out directly in the same reaction medium resulting from the process of the invention, without further purification of the carbinol. Besides being more economical, this direct alkylation avoids racemisation of the compound of formula (II) during workup of the addition reaction according to the present invention, which has been observed under certain reaction conditions.

According to a further aspect, the present invention is directed to a process for the preparation of an enantiomerically enriched compound of formula V 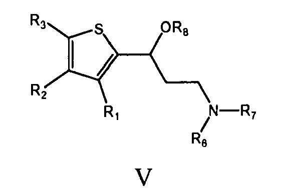 wherein
R₁, R₂, R₃, R₆ and R₇ are as defined above;
R₈ is selected from hydrogen, lower alkyl optionally substituted by halo, hydroxy, alkoxy, alkyloxymethyl, ethers, carboxy, cyano, carbonyl, acyl, alkoxycarbonyl, amino, nitro, mercapto or alkylthio;, hydroxyl protecting group or aryl optionally substituted by hydroxy, mercapto, halo, alkyl, phenyl, alkoxy, haloalkyl, nitro, dialkylamino, aminoalkyl, acyl or alkoxycarbonyl;
or a pharmaceutically acceptable salt, complex or solvate thereof, which comprises,
a) an enantioselective addition reaction to a compound of formula III 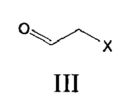 wherein X has the same meaning as above;
   of a thienyl zinc reagent optionally susbtituted at 2, 3, 4 or 5 position of the thienyl ring by halogen, a lower alkyl or aryl group, in the presence of a chiral ligand to yield a compound of formula II 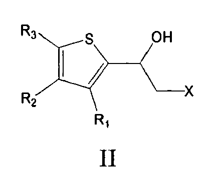 wherein,
   R₁, R₂, R₃ and X are as defined above;
   and further comprises in any order one or more of the steps selected from the group consisting of:
b) alkylation of the hydroxyl group;
c) amide formation;
d) reduction of amide to amine;
e) nucleophilic substitution;
e) hydroxyl, amide or amine protection;
f) hydroxyl , amide or amine deprotection;
g) salt, complex or solvate formation.

Preferably, the compound of formula V is an enantiomerically enriched form of N-methyl-N-[3-(naphthalene-1-yloxy)-3-(2-thienyl)propyl]amine or a pharmaceutically acceptable salt, complex or solvate thereof;, more preferably, in the form of a hydrochloride salt.

The term "enantiomerically enriched compound " refers to a compound that has at least one chiral center and in which there is excess of one enantiomer over the other. Thus it does not comprise the racemic mixture. For a mixture of (+)- and (-)-enantiomers, with composition given as the mole or weight fractions *F*(+) and *F*(-) (where *F*(+) + *F*(-) = 1) the enantiomer excess is defined as |*F*(+) - *F*(-)|. (and the percent enantiomer excess by 100|*F*(+) - *F*(-)|). Frequently this term is abbreviated as e.e. Enantiomerically enriched compound means that the e.e is not 0. In the present invention it is preferred that the e.e is above 505, more preferably above 60%, even more preferably above 70% or 80%, and most preferably above 90% or 95%.

The term "lower alkyl" refers to a linear or branched hydrocarbon chain which contains about 1 to 5 carbon atoms such as methyl, ethyl, propyl, isopropyl, butyl, isobutyl, sec-butyl, tert-butyl, pentyl, etc. The alkyl radical may be optionally substituted by one or more substituents such as halo, hydroxy, alkoxy, alkyloxymethyl ethers, carboxy, cyano, carbonyl, acyl, alkoxycarbonyl, amino, nitro, mercapto and alkylthio.

By "thienyl zinc reagent optionally substituted on the thienyl ring", we refer to a thienyl zinc reagent which can be substituted at 2, 3, 4 or 5 position of the thienyl ring by an halogen, a lower alkyl or an aryl group.

"halogen" refers to fluorine, chlorine, bromine or Iodine.

"Aryl" refers to an aromatic hydrocarbon radical such as phenyl, naphthyl or anthracyl. The aryl radical may be optionally substituted by one or more substituents such as hydroxy, mercapto, halo, alkyl, phenyl, alkoxy, haloalkyl, nitro, cyano, dialkylamino, aminoalkyl, acyl and alkoxycarbonyl, as defined herein.

"Aralkyl" refers to an aryl group linked to an alkyl group such as benzyl and phenethyl.

"Heterocyclic" or "heterocycle" refers to a stable 3- to 15- membered ring which consists of carbon atoms and from one to five heteroatoms selected from the group consisting of nitrogen, oxygen, and sulphur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

"Heteroaryl" refers to a heterocyclic group wherein at least one of the rings is an aromatic ring.

"Hydroxyl protecting group" refers to a group that blocks the OH function tor further reactions and can be removed under controlled conditions. The hydroxyl protecting groups are well known in the art, representative protecting groups are silyl ethers such as trimethylsilyl ether, triethylsilyl ether, tert-butyldimethylsilyl ether, tert-butyldiphenylsilyl ether, tri-isopropylsilyl ether, diethylisopropylsilyl ether, thexyldimethylsilyl ether, triphenylsilyl ether, di-tert-butylmethylsilyl ether; alkyl ethers such as methyl ether, tert-butyl ether, benzyl ether, p-methoxybenzyl ether , 3,4-dimethoxybenzyl ether, trityl ether; allyl ether; alkoxymethyl ether such as methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl and related ethers; methylthiomethyl ether; Esters such as acetate ester, benzoate ester; pivalate ester; methoxyacetate ester; chloroacetate ester; levulinate ester; Carbonates such as benzyl carbonate, p-nitrobenzyl carbonate, tert-butyl carbonate, 2,2,2-trichloroethyl carbonate, 2-(trimethylsilyl)ethyl carbonate, allyl carbonate; and sulphates such as SO₃-py. In some cases activating groups such as triflate are also included as protecting groups. Additional examples of hydroxyl protecting groups can be found in reference books such as Greene and Wuts' "Protective Groups in Organic Synthesis", John Wiley & Sons, Inc., New York, 1999.

"Amino protecting group" refers to a group that blocks the NH₂ function for further reactions and can be removed under controlled conditions. The amino protecting groups are well known in the art, representative protecting groups are carbamates and amides such as substituted or unsubstituted or substituted acetates. Also different alkyl moeties may serve as amino protecting groups. Said alkyl groups may optionally be substituted by one or more substituents such as halo, hydroxy, alkoxy, alkyloxymethyl ethers, carboxy, cyano, carbonyl, acyl, alkoxycarbonyl, amino, nitro, mercapto and alkylthio. Additional examples of amino protecting groups can be found in reference books such as Greene and Wuts' "Protective Groups in Organic Synthesis", John Wiley & Sons, Inc., New York, 1999.

"Amido protecting group" refers to a group that blocks the -C(=O)NH₂ function for further reactions and can be removed under controlled conditions. The amido protecting groups are well known in the art, representative protecting groups are carbamates such as substituted or unsubstituted acetates. Also different alkyl moeties may serve as amido protecting groups. Said alkyl groups may optionally be substituted by one or more substituents such as halo, hydroxy, alkoxy, alkyloxymethyl ethers, carboxy, cyano, carbonyl, acyl, alkoxycarbonyl, amino, nitro, mercapto and alkylthio. Additional examples of amido protecting groups can be found in reference books such as Greene and Wuts' "Protective Groups in Organic Synthesis"; John Wiley & Sons, Inc., New York, 1999.

"ester activating group" referrers to a group which increases the reactivity of the ester functionality.

"hydroxyl activating group" referrers to a group which increases the reactivity of the hydroxyl functionality.

References herein to substituted groups in the compounds of the present invention refer to the specified moiety that may be substituted at one or more available positions by one or more suitable groups, e. g., halogen such as fluoro, chloro, bromo and iodo ; cyano; hydroxyl ; nitro ; azido ; alkanoyl such as a C1-6 alkanoyl group such as acyl and the like ; carboxamido ; alkyl groups including those groups having 1 to about 12 carbon atoms or from 1 to about 6 carbon atoms and more preferably 1-3 carbon atoms ; alkenyl and alkynyl groups including groups having one or more unsaturated linkages and from 2 to about 12 carbon or from 2 to about 6 carbon atoms ; alkoxy groups having one or more oxygen linkages and from 1 to about 12 carbon atoms or 1 to about 6 carbon atoms ; aryloxy such as phenoxy ; alkylthio groups including those moieties having one or more thioether linkages and from 1 to about 12 carbon atoms or from 1 to about 6 carbon atoms ; alkylsulfinyl groups including those moieties having one or more sulfinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms ; aminoalkyl groups such as groups having one or more N atoms and from 1 to about 12 carbon atoms or from 1 to about 6 carbon atoms ; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The following examples are included as illustration of the present invention but are not limitative.

### General procedure :

The ligand and the boron reagent were weighed into a 10 mL-vial, a magnetic stir bar was added, and the vial was closed and flushed with argon. The solvent and diethylzinc (as a 1.0 molar solution in hexane) were added and the reaction mixture was stirred for 10 minutes. The vial was brought to the indicated temperature and the aldehyde was added either directly as a solution in toluene or slowly via a syringe pump. After 16 h the reaction mixture was quenched and extracted with 1 N HCl solution, saturated Na₂CO₃-solution and dried over MgSO₄. HPLC analyses were conducted on chiral stationary phases: Chiralcel AD heptane/2-propanol 90/10 for the amide substrate or Chiralcel OD heptane/2-propanol 95/5 for the 3-chloro propionaldehyde substrate. The enantiomeric excess was measured.

| | | | |
|---|---|---|---|
| 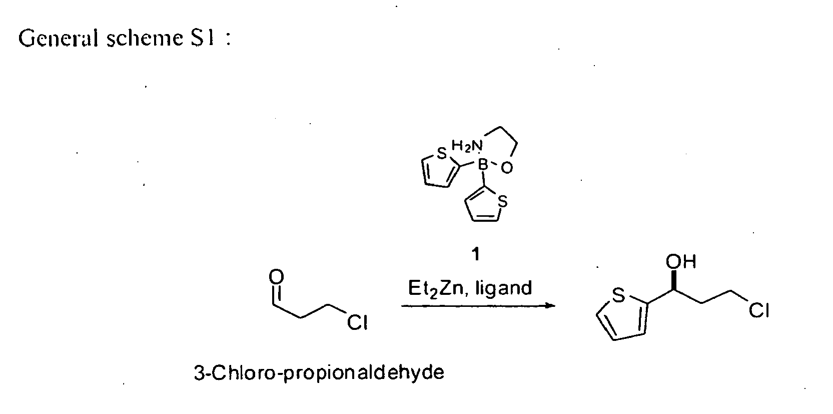 | | | |

| Example | Ligand | conditions | ee |
|---|---|---|---|
| 1 | 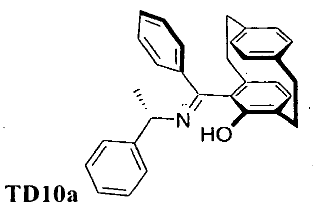 | Borinate (**1**) 60 mg, ligand **TD10a** 10.8 mg, toluene 1 mL, Et₂Zn 1.2 mL (1.2 mmol), aldehyde 23 mg (0,25 mmol in 0.5 mL of toluene), 10°C, 16h | 38% |
| 2 | **TD10a** | as example 1 with 46 mg of borinate **1** | 33% |
| 3 | 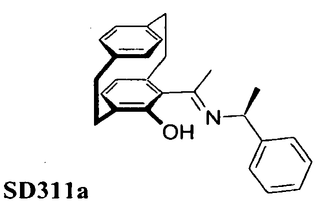 | as example 1 with 7.4 mg of **SD311a** as ligand. | 67% |

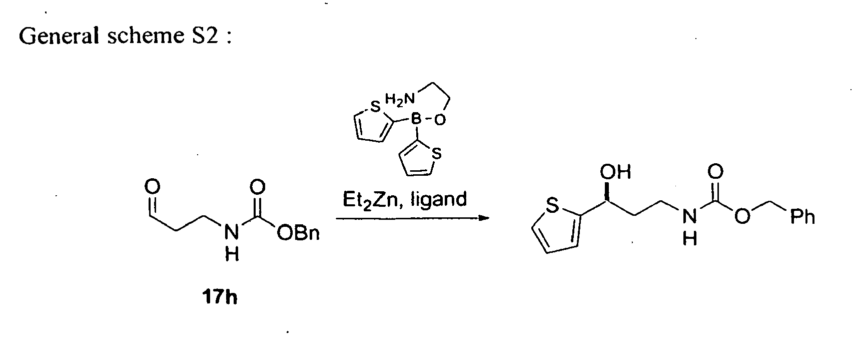

Compound 17h : benzyl 3-oxopropylcarbamate

| Example | Ligand | conditions | ee |
|---|---|---|---|
| 4 | 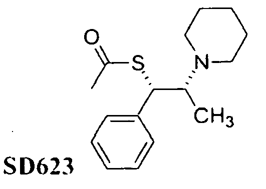 | Borinate (**1**) 63 mg, ligand **SD623** 0.5 mol%, toluene 1 mL, Et₂Zn 1.0 mL (1.0 mmol), slow addition of aldehyde 0.25 mmol (in 0.5 mL of toluene), 10°C, 16h | 20% |
| 5 | **SD623** | as example 4 with 5 mol% of ligand **SD623** | 41% |
| 6 | **SD623** | as example 4 with 10 mol% of ligand **SD623** | 32% |
| 7 | **SD623** | Borinate (**1**) 63 mg, ligand **SD623** 10 mol%, toluene 1.0 mL, Et₂Zn 1.2 mL (1.2 mmol), direct addition of aldehyde 0.25 mmol (in 0.5 mL of toluene), 10°C, 16h | 55% |
| 8 | 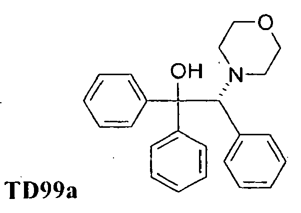 | as example 7 with 10 mol% of ligand **TD99** - | 20% |
| 9 | 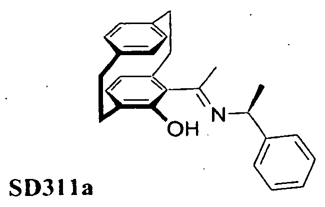 | as example 4 with ligand 7.4 mg **of SD311a** | 41% |

## Claims

1. A process for the preparation of an enantiomerically enriched compound of formula II 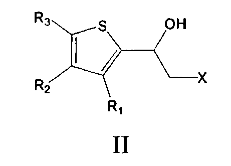 wherein,
R₁, R₂ and R₃ are each independently selected from hydrogen, halogen, alkyl containing 1 to 5 carbon atoms optionally substituted by halo, hydroxy, alkoxy, alkyloxymethyl ethers, carboxy, cyano, carbonyl, acyl, alkoxycarbonyl, amino, nitro, mercapto or alkylthio; or aryl optionally substituted by hydroxy, mercapto, halo, alkyl, phenyl, alkoxy, haloalkyl, nitro, cyano, dialkylamino, aminoalkyl, acyl or alkoxycarbonyl;
X is -C(=O)-Z or -Y, wherein -Y is selected from -CH₂-OR₄, -CH₂-halogen or - CH₂-NR₆R₇;
wherein
Z is selected from -NR₆R₇ or -OR₅, wherein R₅ is selected from hydrogen, alkyl containing 1 to 5 carbon atoms optionally substituted by halo, hydroxy, alkoxy, alkyloxymethyl ethers, carboxy, cyano, carbonyl, acyl, alkoxycarbonyl, amino, nitro, mercapto or alkylthio; or ester activating group;
R₄ is selected from hydrogen, hydroxyl protecting group or hydroxyl activating group;
R₆ and R₇ are each independently selected from hydrogen, amino protecting group, amido protecting group or alkyl containing about 1 to 5 carbon atoms optionally substituted by halo, hydroxy, alkoxy, alkyloxymethyl ethers, carboxy, cyano, carbonyl, acyl, alkoxycarbonyl, amino, nitro, mercapto or alkylthio,
or a pharmaceutically acceptable salt, complex or solvate thereof;
which comprises an enantioselective addition reaction to a compound of formula III 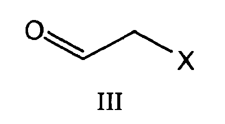 wherein X has the same meaning as above;
with a thienyl zinc reagent optionally substituted at 2, 3, 4 or 5 position of the thienyl ring by a halogen, a lower alkyl or an aryl group, in the presence of a chiral ligand.

2. A process according to claim 1 for the preparation of an enantiomerically enriched compound of formula IIa 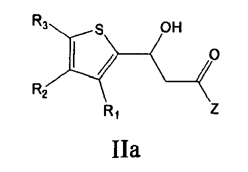 wherein,
R₁, R₂ and R₃ and Z have the same meaning as in claim 1,
or a pharmaceutically acceptable salt, complex or solvate thereof;
which comprises an enantioselective addition reaction to a compound of formula IIIa 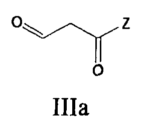 wherein Z has the same meaning as in claim 1;
with a thienyl zinc reagent optionally susbtituted at 2, 3, 4 or 5 position of the thienyl ring by a halogen, a lower alkyl or an aryl group, in the presence of a chiral ligand.

3. A process according to claim 1 for the preparation of an enantiomerically enriched compound of formula IIb 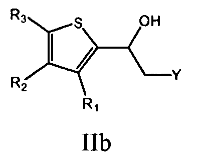 wherein,
R₁, R₂ and R₃ and Y have the same meaning as in claim 1;
or a pharmaceutically acceptable salt, complex or solvate thereof;
which comprises an enantioselective addition reaction to a compound of formula IIIb 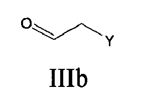 wherein -Y has the same meaning as in claim 1;
with a thienyl zinc reagent optionally susbtituted at 2, 3, 4 or 5 position of the thienyl ring by a halogen, a lower alkyl or an aryl group, in the presence of a chiral ligand.

4. A process according to any of the preceding claims, wherein R₁, R₂ and R₃ are hydrogen.

5. A process according to claim 1 wherein the thienyl zinc reagent is prepared in situ by a transmetallation reaction of a thienylboron reagent with dimethyl-zinc or diethyl-zinc.

6. A process according to claim 5 wherein the thienylboron reagent is selected from thienylboronic acid, trithienylborane or 2-aminoethyl dithienylborinate depicted below:

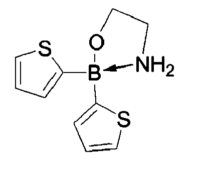

7. A process according to claim 1 wherein the chiral ligand is a N,O-, N,N-, N,S-, N,Se- or O,O-ligand in its enantiomerically pure form.

8. A process according to claim 7 wherein the chiral ligand is a N,O-ligand, most preferably the O is an alcohol.

9. A process according to claim 8 wherein the N,O-ligands are selected from the following compounds: 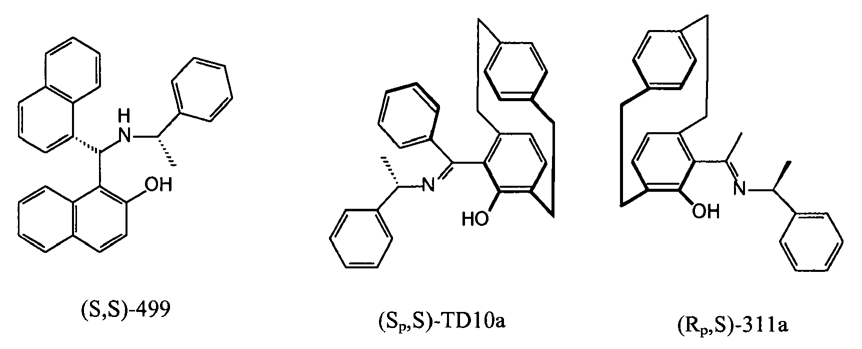 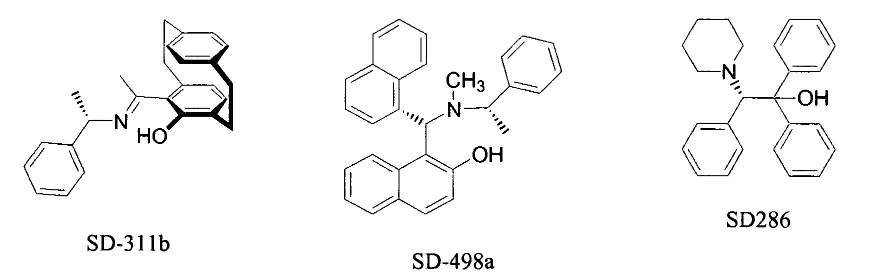 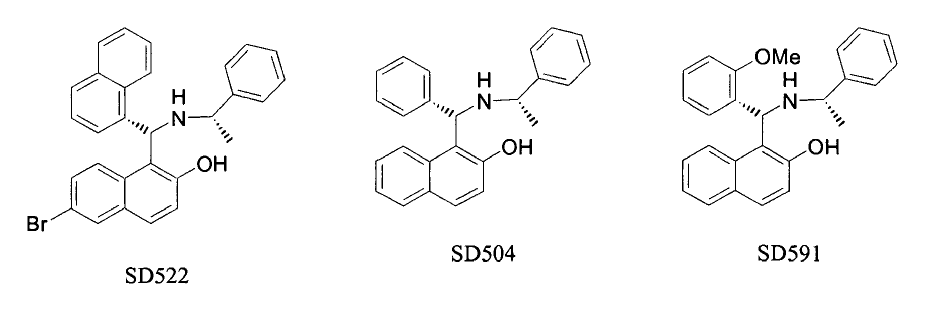 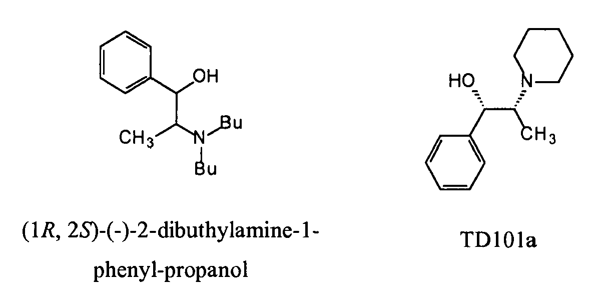 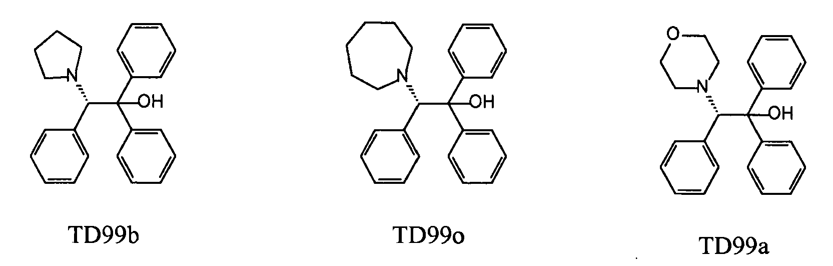 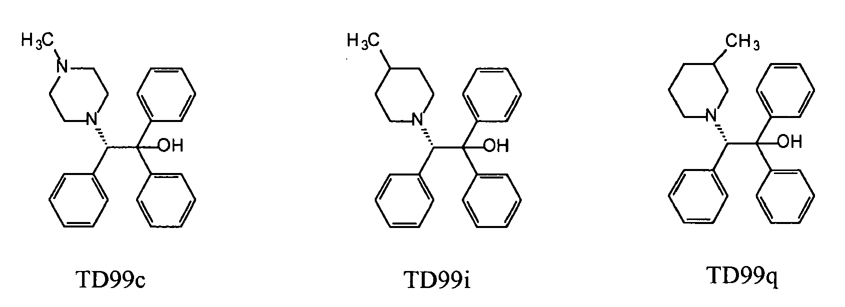 or an enantiomer thereof.

10. A process according to claim 7 wherein the ligand is a N,S-ligand.

11. A process according to claim 10, wherein the ligand is selected from the compounds having the following formulas 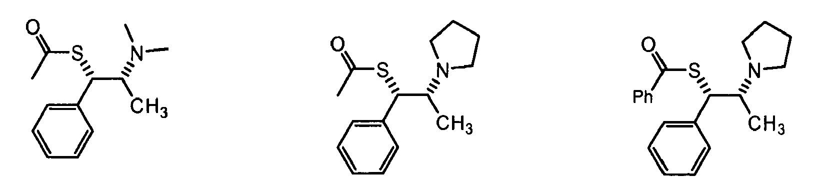 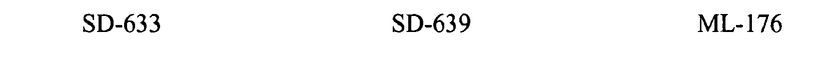 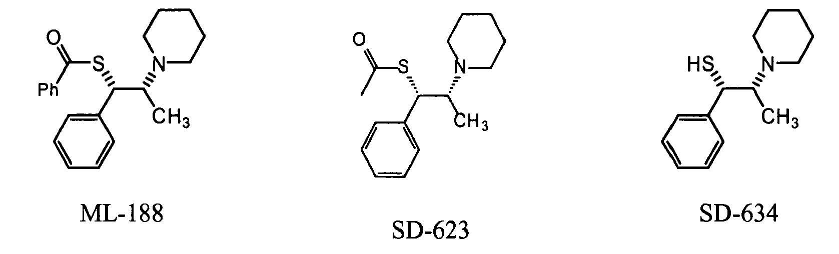 or the enantiomers of the same.

12. A process according to anyone of claims 1-11 wherein the ligands are used in amounts ranged between 0.1 and 20 mol%.

13. A process according to anyone of claims 1-12 wherein the temperature is comprised between -20°C and 20°C, preferably -10°C to 10°C.

14. A process according to anyone of claims 1-13 wherein the aldehyde concentration ranges between 0.01 molar and 2 mo lar.

15. A process according to anyone of claims 1-14 wherein the solvent is non-coordinating, chlorinated or is selected from diethyl ether and methyl-tert-butyl ether.

16. A process according to claim 1 which further comprises an O-alkylation of the enantiomerically enriched compound of formula II.

17. A process according to claim 16, which comprises an O-alkylation with a naphtalene derivative of formula IV 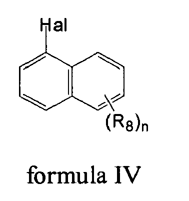 wherein Hal is F, Cl, Br or I; R₈ is alkyl containing about 1 to 5 carbon atoms optionally substituted by halo, hydroxy, alkoxy, alkyloxymethyl ethers, carboxy, cyano, carbonyl, acyl, alkoxycarbonyl, amino, nitro, mercapto or alkylthio; or aryl optionally substituted by hydroxy, mercapto, halo, alkyl, phenyl, alkoxy, haloalkyl, nitro, cyano, dialkylamino, aminoalkyl, acyl or alkoxycarbonyl; and n is comprised between 0 and 7.

18. A process according to any of claims 16 and 17 wherein the O-alkylation is carried out on the product of the process as defined in anyone of claims 1-15, without an intermediate separation or purification step.

19. A process for the preparation of an enantiomerically enriched compound of formula V 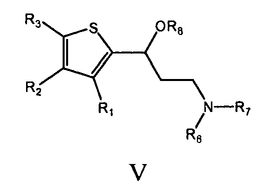 wherein
R₁, R₂, R₃, R₆ and R₇ are as defined in claim 1;
R₈ is selected from hydrogen, alkyl containing about 1 to 5 carbon atoms optionally substituted by halo, hydroxy, alkoxy, alkyloxymethyl ethers, carboxy, cyano, carbonyl, acyl, alkoxycarbonyl, amino, nitro, mercapto or alkylthio; hydroxyl protecting group; or aryl optionally substituted by hydroxy, mercapto, halo, alkyl, phenyl, alkoxy, haloalkyl, nitro, cyano, dialkylamino, aminoalkyl, acyl or alkoxycarbonyl;
or a pharmaceutically acceptable salt, complex or solvate thereof,
which comprises,
a) an enantioselective addition reaction to a compound of formula III 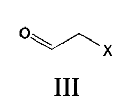
wherein X has the same meaning as in claim 1;
of a thienyl zinc reagent optionally substituted at 2, 3, 4 or 5 position of the thienyl ring by a halogen, a lower alkyl or an aryl group, in the presence of a chiral ligand to yield a compound of formula II 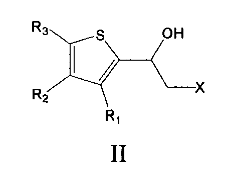 wherein,
R₁, R₂, R₃ and X are as defined in claim 1;
and further comprises in any order one or more of the steps selected from the group consisting of:
b) alkylation of the hydroxyl group;
c) amide formation;
d) reduction of amide to amine;
e) nucleophilic substitution;
e) hydroxyl , amide or amine protection;
f) hydroxyl , amide or amine deprotection;
g) salt, complex or solvate formation.

20. Process according to claim 19, wherein the compound of formula V is an enantiomerically enriched form of N-methyl-N-[3-(naphthalene-1-yloxy)-3-(2-thienyl)propyl]amine or a pharmaceutically acceptable salt, complex or solvate thereof.

21. Process according to any of claims 19 and 20, wherein the compound of formula V is in the form of a hydrochloride salt.

## Patentansprüche

1. Verfahren zur Herstellung einer Enantiomeren-angereicherten Verbindung der Formel (II): 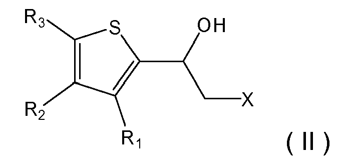 worin:
R₁, R₂ und R₃ jeweils unabhängig voneinander aus Wasserstoff, Halogen, Alkyl mit 1 bis 5 Kohlenstoffatomen, gegebenenfalls substituiert mit Halogen, Hydroxy, Alkoxy, Alkyloxymethylethern, Carboxy, Cyano, Carbonyl, Acyl, Alkoxycarbonyl, Amino, Nitro, Mercapto oder Alkylthio; oder Aryl, gegebenenfalls substituiert mit Hydroxy, Mercapto, Halogen, Alkyl, Phenyl, Alkoxy, Halogenalkyl, Nitro, Cyano, Dialkylamino, Aminoalkyl, Acyl oder Alkoxycarbonyl, ausgewählt sind;
X -C(=O)-Z oder -Y ist, worin -Y aus -CH₂-OR₄, -CH₂-Halogen oder -CH₂-NR₆R₇ ausgewählt ist;
worin
Z aus -NR₆R₇ oder -OR₅ ausgewählt ist, worin R₅ aus Wasserstoff, Alkyl mit 1 bis 5 Kohlenstoffatomen, gegebenenfalls substituiert mit Halogen, Hydroxy, Alkoxy, Alkyloxymethylethern, Carboxy, Cyano, Carbonyl, Acyl, Alkoxycarbonyl, Amino, Nitro, Mercapto oder Alkylthio; oder einer Ester-Aktivierungsgruppe ausgewählt ist;
R₄ aus Wasserstoff, einer Hydroxyl-Schutzgruppe oder einer Hydroxyl-Aktivierungsgruppe ausgewählt ist;
R₆ und R₇ jeweils unabhängig voneinander aus Wasserstoff, einer Amino-Schutzgruppe, einer Amido-Schutzgruppe oder Alkyl mit etwa 1 bis 5 Kohlenstoffatomen, gegebenenfalls substituiert mit Halogen, Hydroxy, Alkoxy, Alkyloxymethylethern, Carboxy, Cyano, Carbonyl, Acyl, Alkoxycarbonyl, Amino, Nitro, Mercapto oder Alkylthio, ausgewählt sind;
oder eines pharmazeutisch annehmbaren Salzes, Komplexes oder Solvats davon;
umfassend eine enantioselektive Additionsreaktion einer Verbindung der Formel (III): 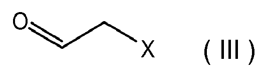 worin X die gleiche Bedeutung wie oben hat;
mit einem Thienyl-Zink-Reagens, das gegebenenfalls an der 2-, 3-, 4- oder 5-Position des Thienylrings mit einem Halogen, einer Niederalkyl- oder einer Arylgruppe substituiert ist, in Gegenwart eines chiralen Liganden.

2. Verfahren gemäss Anspruch 1 zur Herstellung einer Enantiomeren-angereicherten Verbindung der Formel (IIa) : 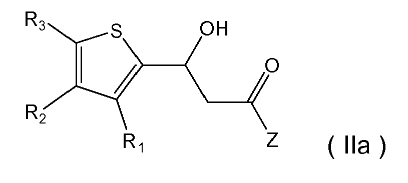 worin:
R₁, R₂, R₃ und Z die gleichen Bedeutungen wie in Anspruch 1 haben,
oder eines pharmazeutisch annehmbaren Salzes, Komplexes oder Solvats davon;
umfassend eine enantioselektive Additionsreaktion einer Verbindung der Formel (IIIa): 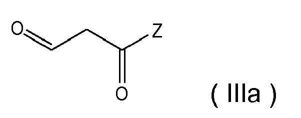 worin Z die gleiche Bedeutung wie in Anspruch 1 hat;
mit einem Thienyl-Zink-Reagens, das gegebenenfalls an der 2-, 3-, 4- oder 5-Position des Thienylrings mit einem Halogen, einer Niederalkyl- oder einer Arylgruppe substituiert ist, in Gegenwart eines chiralen Liganden.

3. Verfahren gemäss Anspruch 1 zur Herstellung einer Enantiomeren-angereicherten Verbindung der Formel (IIb) : 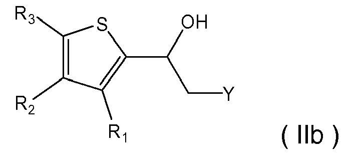 worin:
R₁, R₂, R₃ und Y die gleichen Bedeutungen wie in Anspruch 1 haben;
oder eines pharmazeutisch annehmbaren Salzes, Komplexes oder Solvats davon;
umfassend eine enantioselektive Additionsreaktion einer Verbindung der Formel (IIIb): 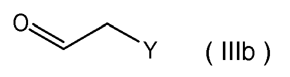 worin -Y die gleiche Bedeutung wie in Anspruch 1 hat; mit einem Thienyl-Zink-Reagens, das gegebenenfalls an der 2-, 3-, 4- oder 5-Position des Thienylrings mit einem Halogen, einer Niederalkyl- oder einer Arylgruppe substituiert ist, in Gegenwart eines chiralen Liganden.

4. Verfahren gemäss irgendeinem der vorhergehenden Ansprüche, worin R₁, R₂ und R₃ Wasserstoff sind.

5. Verfahren gemäss Anspruch 1, wobei das Thienyl-Zink-Reagens durch eine Transmetallierungsreaktion eines Thienylborreagenzes mit Dimethyl-Zink oder Diethyl-Zink in situ hergestellt wird.

6. Verfahren gemäss Anspruch 5, wobei das Thienylborreagens aus Thienylboronsäure, Trithienylboran oder dem nachstehend dargestellten 2-Aminoethyldithienylborinat ausgewählt ist:

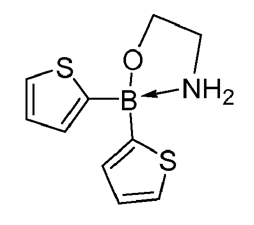

7. Verfahren gemäss Anspruch 1, wobei der chirale Ligand ein N,O-, N,N-, N,S-, N,Se- oder 0,0-Ligand in seiner enantiomer reinen Form ist.

8. Verfahren gemäss Anspruch 7, wobei der chirale Ligand ein N,O-Ligand ist, wobei O am meisten bevorzugt Alkohol ist.

9. Verfahren gemäss Anspruch 8, wobei die N,O-Liganden aus den folgenden Verbindungen ausgewählt sind: 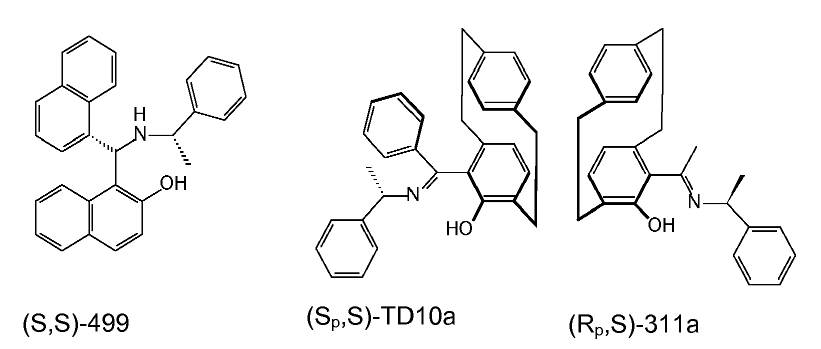 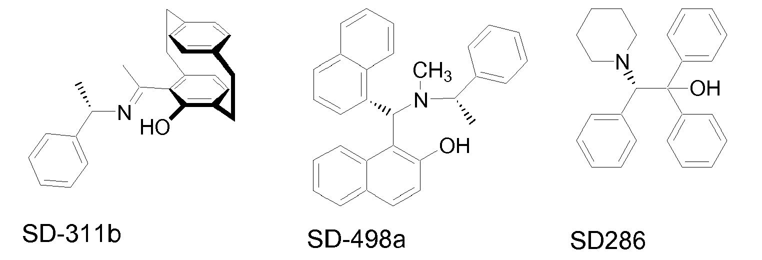 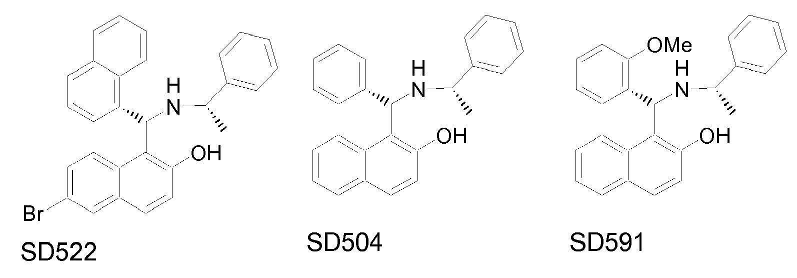 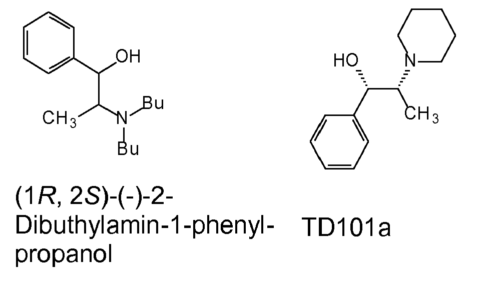 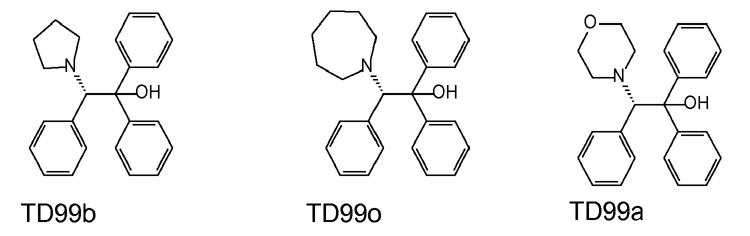 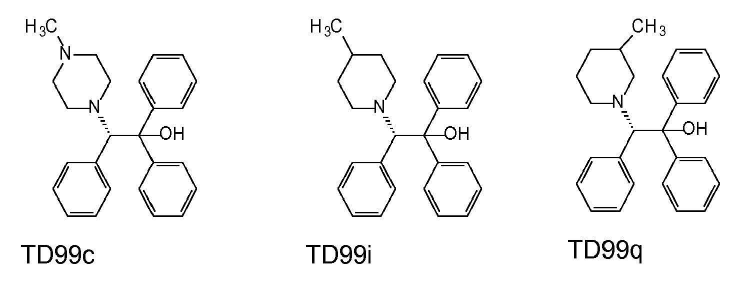 oder ein Enantiomer davon.

10. Verfahren gemäss Anspruch 7, wobei der Ligand ein N,S-Ligand ist.

11. Verfahren gemäss Anspruch 10, wobei der Ligand aus den Verbindungen mit den nachstehenden Formeln ausgewählt ist: 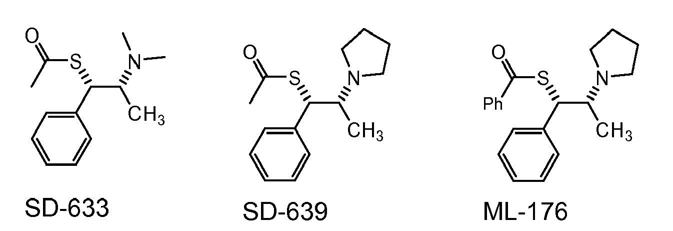 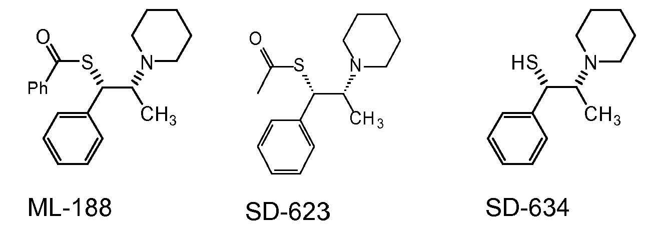 oder die Enantiomere derselben.

12. Verfahren gemäss irgendeinem der Ansprüche 1 bis 11, wobei die Liganden in Mengen zwischen 0,1 und 20 mol-% verwendet werden.

13. Verfahren gemäss irgendeinem der Ansprüche 1 bis 12, wobei die Temperatur zwischen -20 und 20°C, vorzugsweise -10 bis 10°C, umfasst ist.

14. Verfahren gemäss irgendeinem der Ansprüche 1 bis 13, wobei die Aldehydkonzentration zwischen 0,01-molar und 2-molar liegt.

15. Verfahren gemäss irgendeinem der Ansprüche 1 bis 14, wobei das Lösungsmittel nicht-koordinierend, chloriert oder aus Diethylether und Methyl-tert-butylether ausgewählt ist.

16. Verfahren gemäss Anspruch 1, das ferner eine 0-Alkylierung der Enantiomeren-angereicherten Verbindung der Formel (II) umfasst.

17. Verfahren gemäss Anspruch 16, umfassend eine 0-Alkylierung mit einem Naphthalinderivat der Formel (IV): 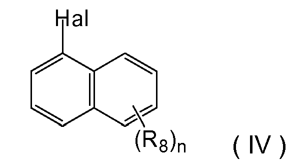 worin Hal F, Cl, Br oder I ist; R₈ Alkyl mit etwa 1 bis 5 Kohlenstoffatomen, gegebenenfalls substituiert mit Halogen, Hydroxy, Alkoxy, Alkyloxymethylethern, Carboxy, Cyano, Carbonyl, Acyl, Alkoxycarbonyl, Amino, Nitro, Mercapto oder Alkylthio; oder Aryl, gegebenenfalls substituiert mit Hydroxy, Mercapto, Halogen, Alkyl, Phenyl, Alkoxy, Halogenalkyl, Nitro, Cyano, Dialkylamino, Aminoalkyl, Acyl oder Alkoxycarbonyl, ist; und n zwischen 0 und 7 liegt.

18. Verfahren gemäss irgendeinem der Ansprüche 16 und 17, wobei die 0-Alkylierung an dem Produkt des Verfahrens wie in irgendeinem der Ansprüche 1 bis 15 definiert ohne Zwischenproduktabtrennung oder Reinigungsstufe durchgeführt wird.

19. Verfahren zur Herstellung einer Enantiomeren-angereicherten Verbindung der Formel (V): 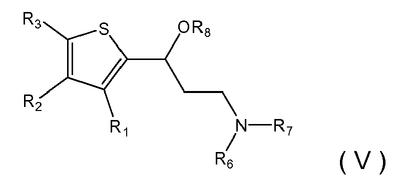 worin:
R₁, R₂, R₃, R₆ und R₇ wie in Anspruch 1 definiert sind;
R₈ aus Wasserstoff, Alkyl mit etwa 1 bis 5 Kohlenstoffatomen, gegebenenfalls substituiert mit Halogen, Hydroxy, Alkoxy, Alkyloxymethylethern, Carboxy, Cyano, Carbonyl, Acyl, Alkoxycarbonyl, Amino, Nitro, Mercapto oder Alkylthio; einer Hydroxyl-Schutzgruppe; oder Aryl, gegebenenfalls substituiert mit Hydroxy, Mercapto, Halogen, Alkyl, Phenyl, Alkoxy, Halogenalkyl, Nitro, Cyano, Dialkylamino, Aminoalkyl, Acyl oder Alkoxycarbonyl, ausgewählt ist;
oder eines pharmazeutisch annehmbaren Salzes, Komplexes oder Solvats davon,
umfassend:
(a) eine enantioselektive Additionsreaktion einer Verbindung der Formel (III): 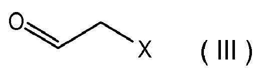 worin X die gleiche Bedeutung wie in Anspruch 1 hat;
eines Thienyl-Zink-Reagenzes, das gegebenenfalls an der 2-, 3-, 4- oder 5-Position des Thienylrings mit einem Halogen, einer Niederalkyl- oder eine Arylgruppe substituiert ist, in Gegenwart eines chiralen Liganden, um eine Verbindung der Formel (II) zu ergeben: 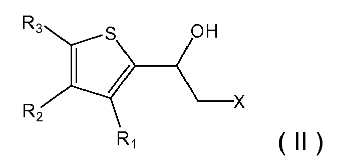 worin:
R₁, R₂, R₃ und X wie in Anspruch 1 definiert sind;
und ferner umfassend, in jeder beliebigen Reihenfolge, eine oder mehrere der Stufen, ausgewählt aus der Gruppe bestehend aus:
(b) Alkylierung der Hydroxylgruppe;
(c) Amidbildung;
(d) Umsetzen des Amids zu Amin;
(e) nukleophiler Substitution;
(e) Schützen von Hydroxyl, Amid oder Amin;
(f) Hydroxyl-, Amid- oder Amin-Entschützung;
(g) Salz-, Komplex- oder Solvatbildung.

20. Verfahren gemäss Anspruch 19, wobei die Verbindung der Formel (V) eine Enantiomeren-angereicherte Form von N-Methyl-N-[3-(naphthalin-1-yloxy)-3-(2-thienyl)propyl]amin oder einem pharmazeutisch annehmbaren Salz, Komplex oder Solvat davon ist.

21. Verfahren gemäss irgendeinem der Ansprüche 19 und 20, wobei die Verbindung der Formel (V) in Form des Hydrochloridsalzes vorliegt.

## Revendications

1. Procédé de préparation d'un composé enrichi en énantiomère de formule II : 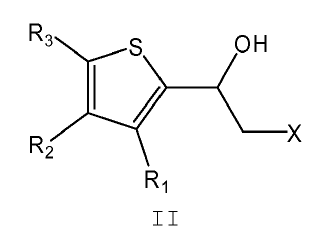 **caractérisé en ce que** :
les groupes R₁, R₂ et R₃ sont chacun choisis indépendamment parmi l'hydrogène, un halogène, un alkyle contenant 1 à 5 atomes de carbone éventuellement substitué par un halogène, un groupement hydroxyle, alcoxyle, éthers d'alkyloxyméthyle, carboxyle, cyano, carbonyle, acyle, alkoxycarbonyle, amino, nitro, mercapto ou alkylthio, ou aryle, ce dernier étant éventuellement substitué par un groupement hydroxyle, mercapto, halogène, alkyle, phényle, alcoxyle, alkyle halogéné, nitro, cyano, dialkylamino, aminoalkyle, acyle ou alkoxycarbonyle,
X est représenté par -C(=O)-Z ou -Y, où Y est choisi parmi les groupements -CH₂-OR₄, -CH₂-halogéné ou -CH₂-NR₆R₇,
**en ce que**
Z est choisi entre -NR₆R₇ et -OR₅, où R₅ est choisi parmi l'hydrogène, un alkyle contenant 1 à 5 atomes de carbone éventuellement substitué par un halogène, un groupement hydroxyle, alcoxyle, éthers d'alkyloxyméthyle, carboxyle, cyano, carbonyle, acyle, alkoxycarbonyle, amino, nitro, mercapto ou alkylthio, ou un groupe d'activation d'ester,
R4 est choisi parmi l'hydrogène, un groupe de protection de l'hydroxyle ou un groupe d'activation de l'hydroxyle,
R₆ et R₇ sont choisis chacun indépendamment parmi l'hydrogène, un halogène, un alkyle contenant 1 à 5 atomes de carbone éventuellement substitué par un halogène, un groupement hydroxyle, alcoxyle, éthers d'alkyloxyméthyle, carboxyle, cyano, carbonyle, acyle, alkoxycarbonyle, amino, nitro, mercapto ou alkylthio,
ou un sel complexe ou solvaté pharmaceutiquement acceptable de ceux-ci,
qui comprend une réaction d'addition énantio-sélective à un composé de formule III : 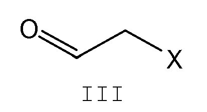 où X a la même signification que ci-dessus,
avec un réactif de thiényle de zinc éventuellement remplacé à la position 2, 3, 4 ou 5 de l'anneau de thiényle par un halogène, un alkyle inférieur ou un groupement aryle, en présence d'un ligand chiral.

2. Procédé selon la revendication 1, pour la préparation d'un composé énantiomèrement enrichi de formule IIa 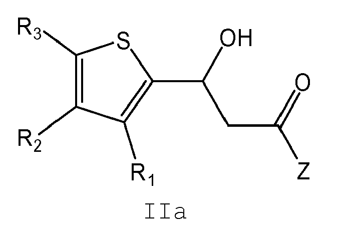 **caractérisé en ce que** les groupes R₁, R₂ et Z ont la même signification que dans la revendication 1,
ou un sel, complexe ou solvaté pharmaceutiquement acceptable de ceux-ci,
qui comprend une réaction d'addition énantiosélective à un composé de formule IIIa 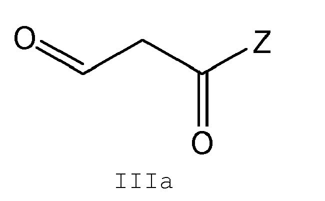 où Z a la même signification que dans la revendication 1, avec un réactif de thiényle de zinc éventuellement remplacé à la position 2, 3, 4 ou 5 de l'anneau de thiényle par un halogène, un alkyle inférieur ou un groupement aryle en présence d'un ligand chiral.

3. Procédé selon la revendication 1 pour la préparation d'un composé énantiomèrement enrichi de formule Iib : 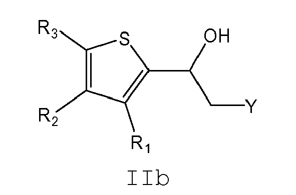 **caractérisé en ce que** les groupes R₁, R₂, R₃ et Y ont la même signification que dans la revendication 1,
ou bien un sel, complexe ou solvaté pharmaceutiquement acceptable de ceux-ci,
qui comprend une réaction d'addition énantiosélective à un composé de formule IIIb : 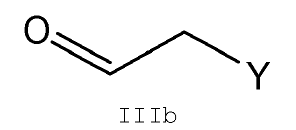 où -Y a la même signification que dans la revendication 1, avec un réactif de thiényle de zinc éventuellement remplacé à la position 2, 3, 4 ou 5 de l'anneau de thiényle par un halogène, un alkyle inférieur ou un groupement aryle en présence d'un ligand chiral.

4. Procédé selon l'une quelconque des revendications précédentes, **caractérisé en ce que** les groupes R₁, R₂ et R₃ sont représentés par l'hydrogène.

5. Procédé selon la revendication 1, **caractérisé en ce que** le réactif de thiényle de zinc est préparé in situ par une réaction de transmétallation d'un réactif de thiényle de bore avec du diméthyle de zinc ou du diéthyle de zinc.

6. Procédé selon la revendication 5, **caractérisé en ce que** le réactif de thiényle de bore est choisi parmi l'acide thiénylborique, le trithiénylborane ou le 2-aminoéthyle dithiénylborinate décrit ci-dessous :

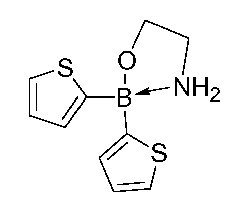

7. Procédé selon la revendication 1, **caractérisé en ce que** le ligand chiral est un ligand N,O-, N,N-, N,S-, N, Se- ou O,O- dans sa forme énantiomèrement pure.

8. Procédé selon la revendication 7, **caractérisé en ce que** le ligand chiral est un ligand N,O-, O étant de préférence un alcool.

9. Procédé selon la revendication 8, **caractérisé en ce que** les ligands N,O- sont choisis parmi les composés suivants : 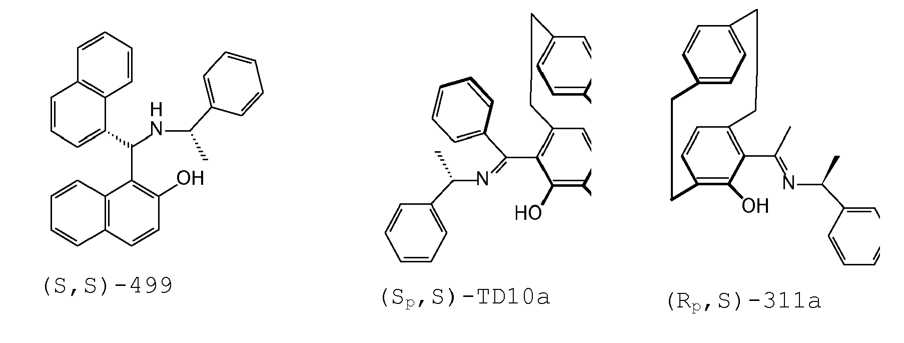 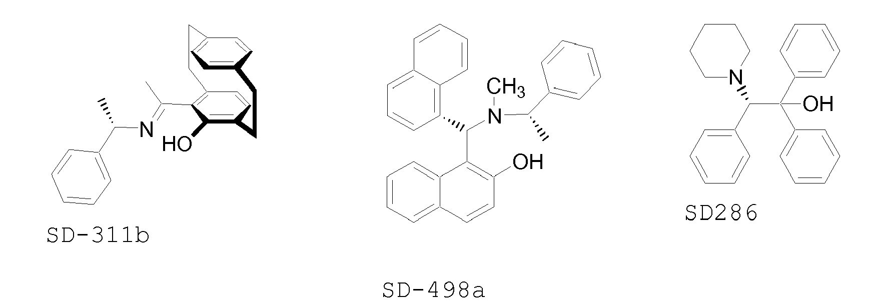 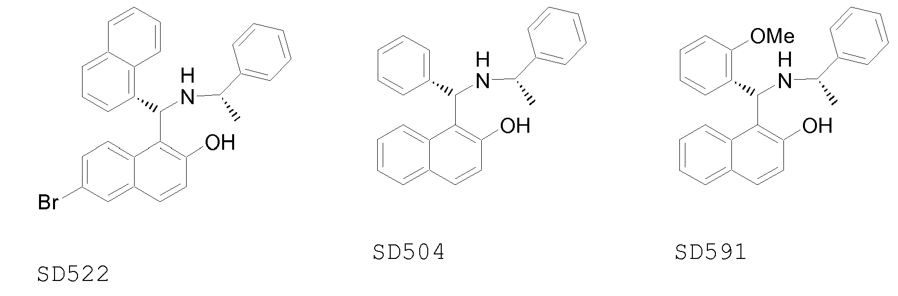 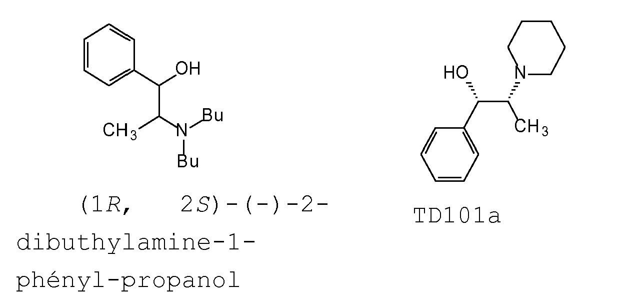 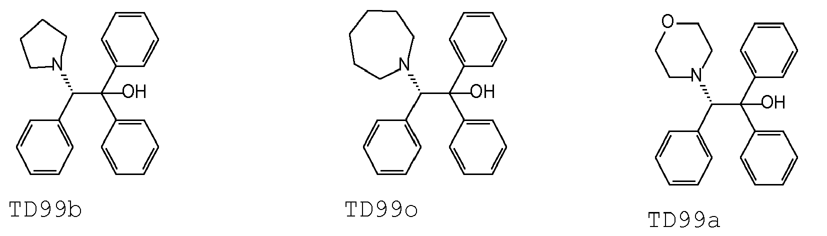 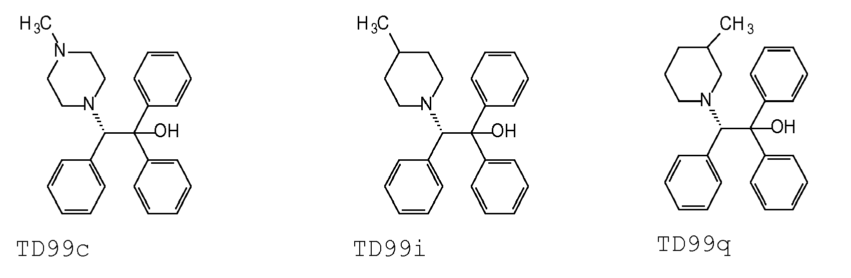 ou un de leurs énantiomères.

10. Procédé selon la revendication 7, **caractérisé en ce que** le ligand est un ligand N,S-.

11. Procédé selon la revendication 10, **caractérisé en ce que** le ligand est choisi parmi les composés ayant les formules suivantes : 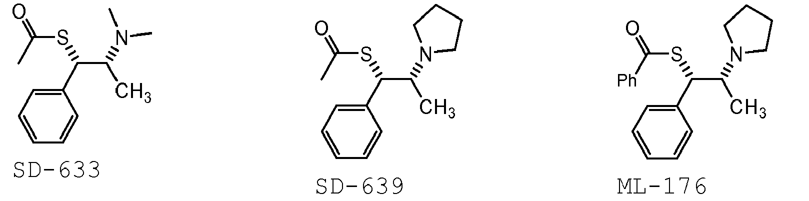 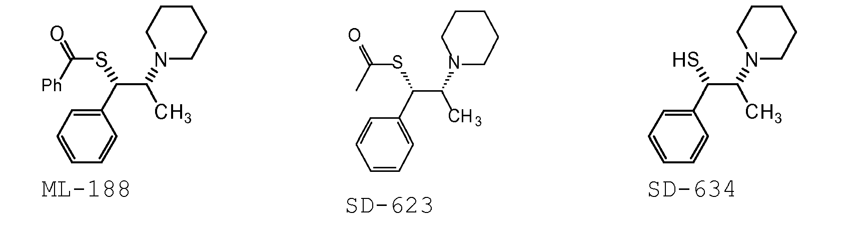 ou un de leurs énantiomères.

12. Procédé selon l'une quelconque des revendications 1 à 11, **caractérisé en ce que** les ligands sont utilisés dans des quantités comprises entres 0,1 et 20% en mol.

13. Procédé selon l'une quelconque des revendications 1 à 12, **caractérisé en ce que** la température est comprise entre -20 et +20°C, de préférence entre -10°C et +10°C.

14. Procédé selon l'une quelconque des revendications 1 à 13, **caractérisé en ce que** la concentration en aldéhyde est comprise entre 0,01 et 2 moles.

15. Procédé selon l'une quelconque des revendications 1 à 14, **caractérisé en ce que** le solvant est non-coordonnant, chloré ou est choisi entre l'éther de diéthyle et l'éther de méthyl-tert-butyle.

16. Procédé selon la revendication 1 qui comprend en outre une 0-alkylation du composé énantiomèrement enrichi de formule II.

17. Procédé selon la revendication 16, qui comprend une 0-alkylation avec un dérivé naphtalénique de formule IV : 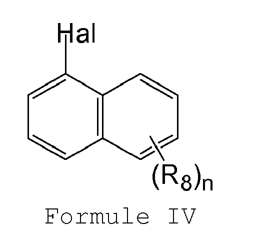 **caractérisé en ce que** le groupe Hal est représenté par F, Cl, Br ou I, R₈ est un alkyle contenant 1 à 5 atomes de carbone éventuellement substitué par un halogène, un groupement hydroxyle, alcoxyle, éther d'alkyloxyméthyle, carboxyle, cyano, carbonyle, acyle, alkoxycarbonyle, amino, nitro, mercapto ou alkylthio, ou aryle éventuellement substitué par un groupe hydroxy, mercapto, halogène, alkyle, phényle, alkoxy, haloalkyle, nitro, cyano, dialkylamino, aminoalkyle, acyle ou de alkoxycarbonyle, et n est compris entre 0 et 7.

18. Procédé selon l'une des revendications 16 et 17, **caractérisé en ce que** l'O-alkylation est réalisée sur le produit du procédé tel que défini dans l'une quelconque des revendications 1 à 15, sans étape intermédiaire de séparation ou de purification.

19. Procédé de préparation d'un composé énantiomèrement enrichi de formule V : 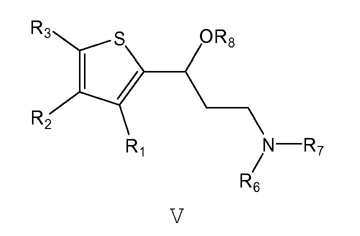 **caractérisé en ce que**
les groupes R₁, R₂, R₃, R₆ et R₇ sont tels que définis dans la revendication 1,
R₈ est choisi entre l'hydrogène, un alkyle contenant 1 à 5 atomes de carbone éventuellement remplacés par un halogène, un groupement hydroxyle, alcoxyle, éthers d'alkyloxyméthyle, carboxyle, cyano, carbonyle, acyle, alkoxycarbonyle, amino, nitro, mercapto ou alkylthio, ou aryle éventuellement remplacé par un groupement hydroxyle, mercapto, halogène, alkyle, phényle, alcoxyle, haloalkyle, nitro, cyano, dialkylamino, aminoalkyle, acyle ou alkoxycarbonyle,
ou un sel complexe ou solvaté de ceux-ci, pharmaceutiquement acceptable,
qui comprend
a) une réaction d'addiction énantiosélective à un composé de formule III 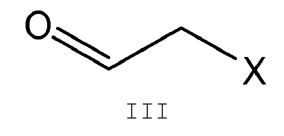
où X a la même signification que dans la revendication 1, d'un réactif de thiényle de zinc éventuellement remplacé à la position 2, 3, 4 ou 5 de l'anneau de thiényle par un halogène, un alkyl inférieur ou un groupement aryle, en présence d'un ligand chiral pour produire un composé de formule II 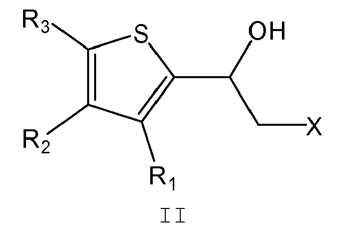 où les groupes R₁, R₂, R₃ et X sont tels que définis dans la revendication 1,
et comprend en outre dans n'importe quel ordre une ou plusieurs des étapes choisies dans le groupe composé de :
b) l'alkylation du groupement hydroxyle,
c) la formation d'amide,
d) la réduction de l'amide en amine,
e) la substitution nucléophile,
e) la protection du groupement hydroxyle, amine ou amide,
f) l'élimination de la protection du groupement hydroxyle, amide ou amine
g) formation du sel, du complexe ou du composé solvaté.

20. Procédé selon la revendication 19, **caractérisé en ce que** le composé de formule V est une forme énantiomère enrichie de N-méthyl-N-[3-(naphthalène-1-yloxy)-3-(2-thiényl)propyl]- amine ou un sel, complexe ou solvaté, pharmaceutiquement acceptable de celui-ci.

21. Procédé selon l'une quelconque des revendications 19 et 20, **caractérisé en ce que** le composé de formule V est sous forme de sel de chlorhydrate.
